(19) Europäisches Patentamt
European Patent Office
Office européen des brevets

(11) Veröffentlichungsnummer: **0 045 720**
**B1**

(12) # EUROPÄISCHE PATENTSCHRIFT

(45) Veröffentlichungstag der Patentschrift: **24.09.86**

(21) Anmeldenummer: **81810305.3**

(22) Anmeldetag: **27.07.81**

(51) Int. Cl.⁴: **C 08 F 220/60**, C 08 F 220/34, C 08 F 220/56, A 61 K 7/06 // (C08F220/60, 220:56, 246:00), (C08F220/56, 220:60, 220:34, 246:00), (C08F220/34, 220:56, 246:00)

(54) **Quaternäre, copolymere, hochmolekulare Ammoniumsalze auf Acrylbasis, deren Herstellung und Verwendung in kosmetischen Mitteln.**

(30) Priorität: **01.08.80 CH 5876/80**

(43) Veröffentlichungstag der Anmeldung: **10.02.82 Patentblatt 82/6**

(45) Bekanntmachung des Hinweises auf die Patenterteilung: **24.09.86 Patentblatt 86/39**

(84) Benannte Vertragsstaaten: **BE CH DE FR GB IT LI NL**

(56) Entgegenhaltungen:
**DE-A-1 961 101**
**DE-A-2 756 697**
**FR-A-2 147 067**
**FR-A-2 206 336**
**FR-A-2 295 049**
**FR-A-2 390 983**
**US-A-3 996 146**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Strasilla, Dieter, Dr., Efringerstrasse 26, D-7858 Weil am Rhein (DE)**
Erfinder: **Meindl, Hubert, Dr., Hungerbachweg 58, CH- 4125 Riehen (CH)**
Erfinder: **Moldovanyi, Laszlo, Dr., Austrasse 78, CH- 4051 Basel (CH)**
Erfinder: **Fearnley, Charles, Dr., Wettsteinanlage 50, CH- 4125 Riehen (CH)**

**EP 0 045 720 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft hochmolekulare, copolymere, quaternäre Ammoniumsalze auf Acrylbasis, die den Rest eines anionischen Tensids oder eines anionischen, zwitterionischen Tensids als Anion aufweisen. Das Molekulargewicht von mindestens 5 Gewichtsprozent des erfindungsgemässen Copolymerisats beträgt 10 bis 10. Die erfindungsgemässen Copolymerisate finden in kosmetischen, insbesondere haarkosmetischen Mitteln Verwendung.

Aus FR-A 2 206 336 sind copolymere, quaternäre Ammoniumsalze auf Acrylbasis bekannt, die jedoch ein Halogenid, Acetat, Methosulfat oder Tosylat als Anion und ein Molekulargewicht von etwa 10 aufweisen und bei der Papierherstellung, bei der Abwasserreinigung, bei photographischen Systemen und als Bodendünger Verwendung finden.

Aus FR-A 2 295 049 sind copolymere, quaternäre Ammoniumsalze auf Acrylbasis bekannt, die jedoch ein Halogenid, Sulfat oder Acetat als Anion aufweisen. Das Molekulargewicht dieser bekannten Copolymerisate ist nicht spezifiziert und die bekannten Copolymerisate finden als Entwässerungsmittel von Klärschlamm Verwendung.

Aus DE-A 1 961 101 sind copolymere, quaternäre Ammoniumsalze auf Acrylbasis bekannt, die jedoch ein Halogenid oder Methosulfat als Anion aufweisen. Das Molekulargewicht dieser bekannten Copolymerisate ist nicht spezifiziert und die bekannten Copolymerisate finden als Verdickungs- und Auflockungsmittel Verwendung.

Aus US-A 3 996 146 sind copolymere, quaternäre Ammoniumsalze auf Acylbasis bekannt, die den Rest von mindestens zwei anionischen Tensiden als Anion aufweisen. Das Molekulargewicht dieser bekannten Copolymerisate ist nicht spezifiziert. Die bekannten Copolymerisate finden in haarkosmetischen Mitteln Verwendung, wobei jedoch ein amphoteres Tensid ohne überschüssige, negative Ladungen zwingend mitverwendet werden muss.

Es wurde nun gefunden, dass die erfindungsgemässen Ammoniumsalze aufgrund ihrer Anteile von mindestens 5 Gew.% im hochmolekularen Bereich von $10^7$ bis $10^9$ bei ihrer Verwendung in haarkosmetischen Mitteln in Abwesenheit von amphoteren Tensiden ausgezeichnete Konditioniereffekte, insbesondere eine erhöhte Nasskämmbarkeit des behandelten Haares bewirken.

Somit sind Gegenstand der vorliegenden Erfindung in wässrigen Tensidsystemen lösliche oder mikroemulgierbare, copolymere, quaternäre Amnoniumsalze, die dadurch gekennzeichnet sind, dass sie eine Molekulargewichtsverteilung von $10^4$ bis $10^9$ aufweisen, wobei das Molekulargewicht von mindestens 5 Gewichtsprozent des Copolymerisats $10^7$ bis $10^9$ beträgt, und in beliebiger Reihenfolge durchschnittlich 5 bis 80 Mol% wiederkehrende Strukturelemente der Formel

(1) 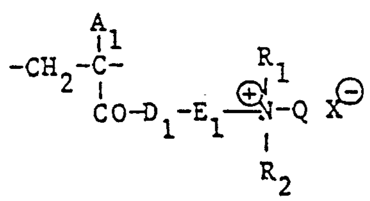

durchschnittlich 10 bis 95 Mol% wiederkehrende Strukturelemente der Formel

(2) 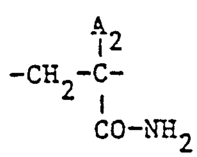

und

0 bis durchschnittlich 10 Mol% wiederkehrende Strukturelemente der Formeln

(3) 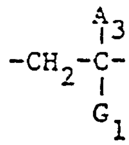

und gegebenenfalls

(4) 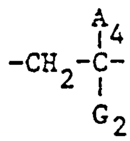

enthalten, worin $A_1$, $A_2$, $A_3$ und $A_4$ je Wasserstoff oder Methyl, $G_1$ und $G_2$ voneinander verschieden sind und je -CN, -COOH oder

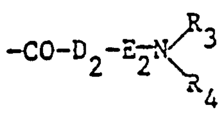,

$D_1$ und $D_2$ je Sauerstoff oder -NH, $E_1$ und $E_2$ je Alkylen mit bis 4 Kohlenstoffatomen, das gegebenenfalls durch Hydroxyl substituiert ist, $R_1$, $R_2$, $R_3$ und $R_4$ je Methyl oder Aethyl, Q Alkyl oder Hydroxyalkyl mit 1 bis 4 Kohlenstoffatomen oder Benzyl und $X^{\ominus}$ den Rest mindestens eines anionischen Tensids oder eines anionischen, zwitterionischen Tensids bedeuten.

Weitere Gegenstände der Erfindung sind das Herstellungsverfahren für die vorstehend angegebenen copolymeren Ammoniumsalze, deren Verwendung in kosmetischen Mitteln, die kosmetischen Mittel (Haarkosmetika), welche die erfindungsgemässen Ammoniumsalze enthalten und deren Applikationsverfahren, insbesondere Verfahren zum Behandeln von Haar.

Die erfindungsgemässen Ammoniumsalze zeichnen sich dadurch aus, dass sie durch Wasser-in-Oel Emulsionspolymerisation oder Lösungspolymerisation eines quaternären Ammoniumsalzes der Acrylsäurereihe und mindestens eines weiteren Comonomers auf Acrylbasis, Isolierung des Copolymerisats und Umsetzung mit einem anionischen, gegebenenfalls zwitterionischen Tensid unter Ionenaustausch erhältlich sind.

Durch die Wasser-in-Oel Emulsionspolymerisation, auch inverse Emulsionspolymerisation genannt bzw. die Lösungspolymerisation, erreicht man den hohen Molekulargewichtsbereich für die erfindungsgemässen Copolymerisate von $10^7$ bis $10^9$, deren Anteil vorzugsweise 5 bis 60 und insbesondere 20 bis 50 Gewichtsprozent der Copolymerisate innerhalb der breiten Molekulargewichtsverteilung von 10 bis 10 ausmacht.

Besonders bevorzugte Polymere enthalten 30 bis 45 Gewichtsprozent Anteile im Molekulargewichtsbereich von $10^7$ bis $10^9$ und weniger als 15 Gewichtsprozent Anteile im Molekulargewichtsbereich kleiner als $10^5$.

Der Anteil an Strukturelementen der Formel (1) in den Copolymerisaten, auch Quatgehalt genannt, bildet neben der Molekulargewichtsverteilung ein weiteres, wesentliches Kennzeichen der erfindungsgemässen Ammoniumsalze, welche durchschnittlich etwa 5 bis 80, vorzugsweise 6 bzw. 9 bis 40 und insbesondere 10 bis 30 Mol% Strukturelemente der Formel (1), durchschnittlich etwa 10 bis 95, vorzugsweise 50 bis 90 Mol% Strukturelemente der Formel (2) und etwa 0 bis 10 Mol% Strukturelemente der Formel (3) und gegebenfalls (4), d.h. (3) und/oder (4) enthalten. Vorzugweise enthalten die Copolymerisate keine Strukturelemente de Formeln (3) und /4). Falls jedoch solche Strukturelemente vorhanden sind, weisen die Copolymerisate etwa 1 bis 8 Mol% Strukturelemente der Formeln (3) und/oder (4), im speziellen je 1 bis 4 Mol% der Formeln (3) und (4) auf.

Als bevorzugte Bedeutungen für die Symbole der Formel (1) gelten für $A_1$ Methyl, für $D_1$ Sauerstoff, für $E_1$ unsubstituiertes n-Propyl oder unsbesondere unsubstituiertes Aethylen, für $R_1$ und $R_2$ Methyl und für Q unsubstituiertes n-Propyl, vorzugsweise Aethyl oder insbesondere Methyl. $A_2$ in Formel (2) steht vorzugsweise für Wasserstoff. Falls Strukturelemente der Formel (3) für sich allein, d.h. in Abwesenheit von Strukturelementen der Formel (4), vorhanden sind, gelten als bevorzugte Bedeutungen für die Symbole de Formel (3) Methyl für $A_3$ und

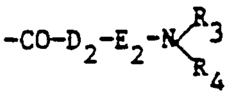

für $G_1$, wobei $D_2$, $E_2$, $R_3$ und $R_4$ die gleichen bevorzugten Bedeutungen haben wie für $D_1$, $E_1$, $R_1$ und $R_2$ vorstehend angegeben. Falls Strukturelemente der Formel (4) zusätzlich zu den Strukturelementen der Formel (3) vorhanden sind, bedeuten in Formel (4) $A_4$ vorzugsweise Wasserstoff und $G_2$ vorzugsweise -CN und insbesondere -COOH.

Falls $X^{\ominus}$ in Formel (1) für den Rest eines anionischen, jedoch nicht zwitterionischen Tensids steht, handelt es sich vorzugsweise um Reste oberflächenaktiver Sarkosinate, Sulfate, z.B. Alkylsulfate, Alkyläthersulfate, Alkylamidsulfate, Alkylamidäthersulfate, Alkylarylpolyäthersulfate oder Monoglyceridsulfate, Sulfonate, z.B. Alkylsulfonate, Alkylamidsulfonate, Alkylarylsulfonate, α-Oelfinsulfonate oder Sulfobernsteinsäurederivate, z.B. Alkylsulfosuccinate, Alkyläthersulfosuccinate, Alkylamidsulfosuccinate, Alkylamidpolyäthersulfosuccinate oder Alkylsulfosuccinamide, ferner auch um Reste von fluorierten Tensiden oder Phosphattensiden, wie z.B. Alkyl- oder Alkylätherphosphaten. In Frage kommen z.B. für $X^{\ominus}$ der Rest eines Sarkosinats der Formel

(5) 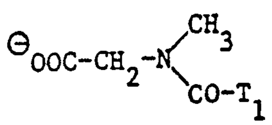 ,

worin $T_1$ Alkyl oder Alkenyl mit 7 bis 21, vorzugsweise 11 bis 17 Kohlenstoffatomen bedeutet.

Hierbei leiten sich die Reste $T_1CO$- von den entsprechenden gesättigten oder umgesättigten Fettsäuren mit 8 bis 22, vorzugsweise 12 bis 18 Kohlenstoffatomen ab. Ala Beispiele der entsprechenden Fettsäuren seien Capryl-, Caprin-, Arachin- und Behensäure, insbesondere Laurin-, Myristin-, Palmitin- und Stearinsäure oder Myristolein-, Palmitolein-, Elaeostearin, Clupanodonsäure, insbesondere Oel-, Elaidin-, Eruka-, Linol- und Linolensäure genannt. Alkyl- und Alkenylreste für $T_1$, die sich von technischen Gemischen der genannten gesättigten und/oder ungesättigten Fettsäuren ableiten, sind besonders bevorzugt. Als spezifische Vertreter von 8arkoainatresten für $X^{\ominus}$ seien vor allem die Reste der Formeln

(6) 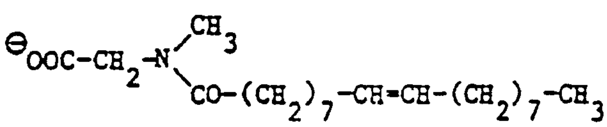

und insbesondere

(7) 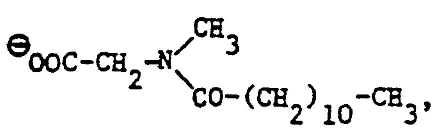

genannt.

Falls $X^{\ominus}$ einen Sulfatrest (von Alkylsulfaten, sowie von Aether-, Ester-, Amid- oder Aminosulfaten) bedeutet, kommen z.B. insbesondere Reste der Formeln

(8) $T_1-O-(CH_2-CH_2-O)_{p-1}-SO_3^{\ominus}$ ,

(9) 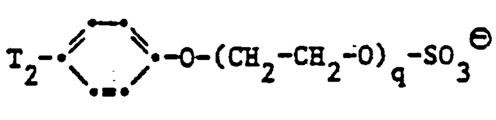 ,

(10) $T_1-CO-NH-(CH_2)_r-O-SO_3^{\ominus}$ oder

(11) $T_1-CO-NH-(CH_2-CH_2-O)_p-SO_3^{\ominus}$

in Betracht, worin $T_1$ die angegebenen Bedeutungen hat und $T_2$ Alkyl mit 6 bis 14 Kohlenstoffatomen, p eine ganze Zahl von 1 bis 50, q eine ganze Zahl von 6 bis 12 und r eine ganze Zahl von 2 bis 6 bedeuten.

$T_2$ steht vorzugsweise für n-Hexyl, i-Octyl, n-Octyl, n-Nonyl, n-Decyl, ferner für Lauryl und Meristyl.

Als spezifische Vertreter von Sulfatresten seien vor allem die Reste der Formel

(12) $^{\ominus}O_3S-O-(CH_2)_{2-4}-NH-CO-(CH_2)_{11}-CH_3$,

(13) $^{\ominus}O_3S-(O-CH_2-CH_2)_{8-10}-NH-CO-(CH_2)_{11}-CH_3$,

(14) 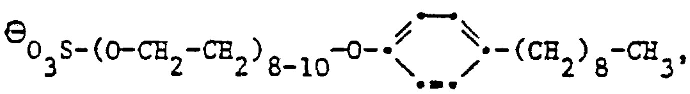 insbesondere

(15) $^{\ominus}O_3S-(O-CH_2-CH_2)_2-O-(CH_2)_{11}-CH_3$ und

(16) $^{\ominus}O_3S-O-(CH_2)_{11}-CH_3$

genannt.

Ist $X^{\ominus}$ ein Sulfatrest, so kommen Reste der Formeln

(17) 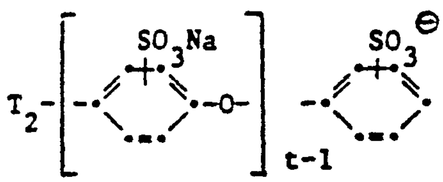 ,

(18) 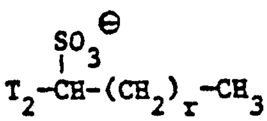 ,

(19) $T_2-COO-CH_2-CH_2-SO_3^{\ominus}$,

(20) 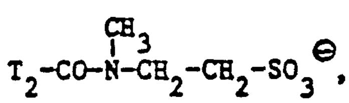

(21) 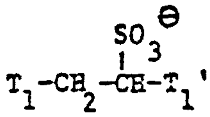 ,

(22) $T_1-CH{=}CH-SO_3^{\ominus}$, oder

(23) 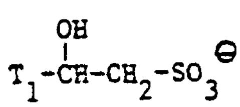

in Betracht, worin $T_1$, $T_2$ und r die angegebenen Bedeutungen haben, $T_1'$ die für $T_1$ angegebenen Bedeutungen hat, wobei $T_1$ und $T_1'$ gleich oder voneinander verschieden sind und t 1 oder 2 bedeutet.

Als spezifische Vertreter solcher Sulfonatreste als Definition von $X^\ominus$ seien vor allem die Reste der Formeln

(24) $CH_3-(CH_2)_{11}-\underset{|}{\overset{SO_3^\ominus}{C}}H-(CH_2)_{2-4}-CH_3$,

(25) $^\ominus O_3S-(CH_2)_2-OOC-(CH_2)_{10}-CH_3$,

(26) $^\ominus O_3S-(CH_2)_2-N\begin{matrix}CH_3\\CO-(CH_2)_7-CH{=}CH-(CH_2)_7-CH_3\end{matrix}$, insbesondere

(27) $^\ominus O_3S-(CH_2)_2-N\begin{matrix}CH_3\\CO-(CH_2)_{10}-CH_3\end{matrix}$ und

(28) 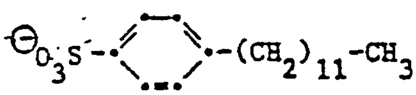

erwähnt.

Bedeutet $X^\ominus$ den Rest eines Sulfo-bernsteinsäurederivats, so entspricht $X^\ominus$ z.B. einer der Formeln

(29) $T_2-OOC-\underset{SO_3^\ominus}{\underset{|}{C}H}-CH_2-COO-[T_2']_{t-1}-[Na]_{2-t}$ ,

(30) $T_1-O-(CH_2-CH_2-O)_{p-1}-OC-CH_2-\underset{SO_3^\ominus}{\underset{|}{C}H}-COONa$ ,

(31) $T_1-CO-NH-CH_2-CH_2-OOC-CH_2-\underset{SO_3^\ominus}{\underset{|}{C}H}-COONa$ ,

(32) 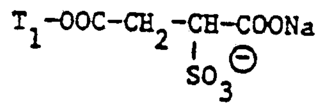 ,

(33) 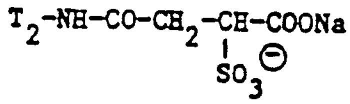 ,

(34) 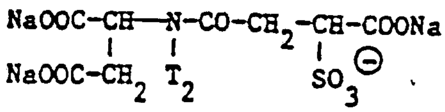 oder

(35) 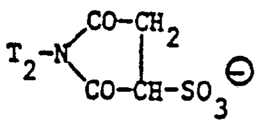 ,

worin $T_1$, $T_2$, p und t die angegebenen Bedeutungen haben und $T_2'$ die für $T_2$ angegebenen Bedeutungen hat, wobei $T_2$ und $T_2'$ gleich oder voneinander verschieden sind.

Als spezifische Vertreter solcher Reste für X ⊖ seien die Reste der Sulfobernsteinsäurederivate der Formeln

(36) 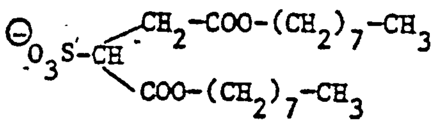 ,

(37) 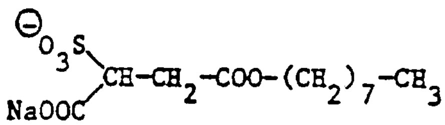 ,

(38) 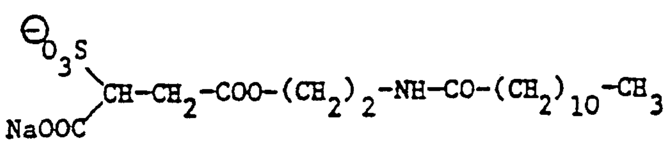 ,

(39) 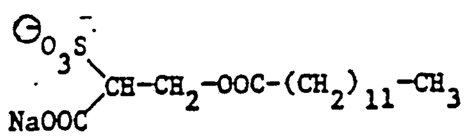 ,

(40) 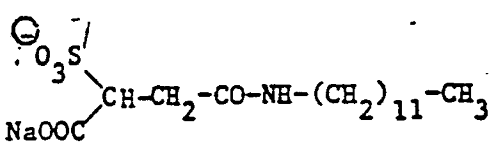 ,

(41) 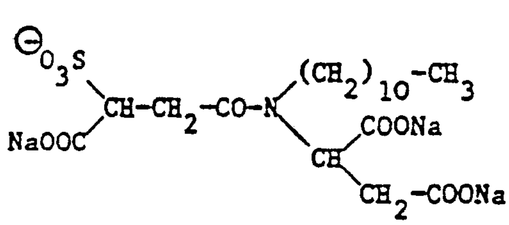 ,

(42) 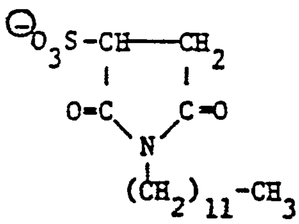 und insbesondere

(43) 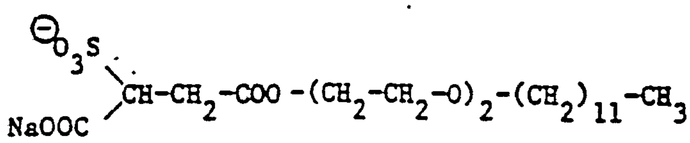

erwähnt.

Als Rest eines fluorierten Tensids als mögliche Bedeutung für $X^{\ominus}$ kommen vor allem Reste der Formel

(44) 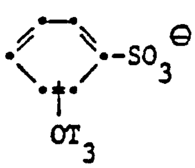

in Frage, worin $T_3$ perfluoriertes Alkyl oder Alkenyl mit 6 bis 14 Kohlenstoffatomen bedeutet.

Der perfluorierte Tensidrest der Formel

(45) $^{\ominus}O_3S-C_6H_4OC_{10}F_{19}$

stellt ein mögliches Beispiel solcher Reste dar.

Ist $X^{\ominus}$ ein Phosphattensidrest, so kommen vor allem Tensidreste der Formeln

(46) 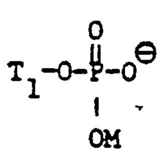 ,

(47) 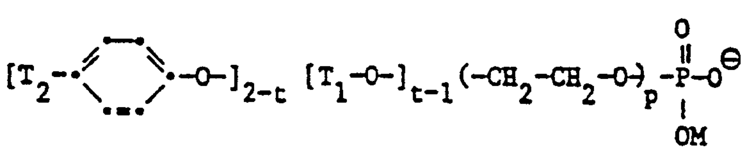

oder

(48) 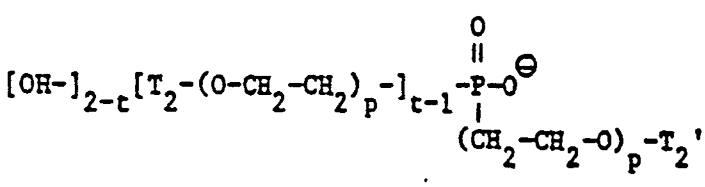

ist, worin M Wasserstoff, Ammonium, ein Alkalimetall oder Alkyl mit 1 bis 3 Kohlenstoffatomen ist, $T_1$, $T_2$, $T_2'$, p und t die angegebenen Bedeutungen haben, p' die für p angegebene Bedeutung hat und p und p' gleich oder voneinander verschieden sind.

Als Beispiele solcher Phosphattensidreste seien die Reste der Formeln

(49) 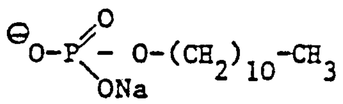 ,

(50) 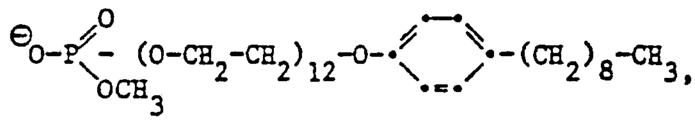 ,

(51) 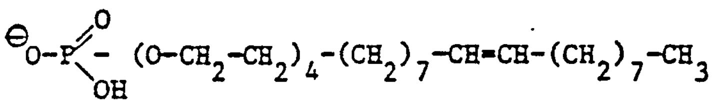 ,

(52) 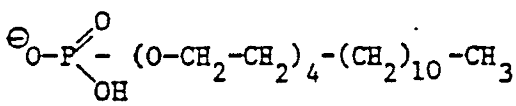 und

(53) 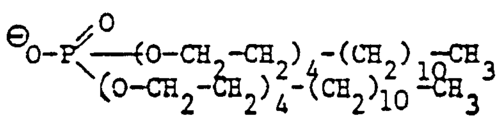

und deren Gemische, z.B. technische Gemische von Phosphattensidresten der Formeln (52) und (53) genannt.

Bei den Resten $X^{\ominus}$ in der Bedeutung von Resten zwitterionisch anionischer Tenside in Formel (1) handelt es sich um Tensidreste, die in der Regel eine positive und zwei negative Ladungen aufweisen, d.h. um ampholytische Tensidreste, die stets einen Ueberschuss sn negativen Ladungen aufweisen. Als Reste solcher zwitterionisch anionischer Tenside kommen vor allem Reste von oberflächenaktiven N-alkyl-α-iminodipropionaten und insbesondere von in 2-Stellung alkylsubstituierten Imidazoliniumdicarbonsäurederivaten in Betracht.

Reste von alkylsubstituierten Iminodipropionaten entsprechen vorzugsweise der Formel

(54) 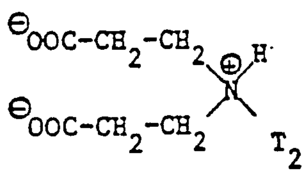 ,

worin $T_2$ die angegebene Bedeutung hat.

Als spezifisches Beispiel sei der Rest der Formel

(55) 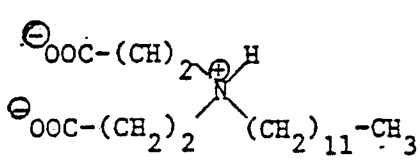

genannt.

Bei den bevorzugten Imidazoliniumresten der angegebenen Art handelt es sich vor allem um Reste der Formel

(56) 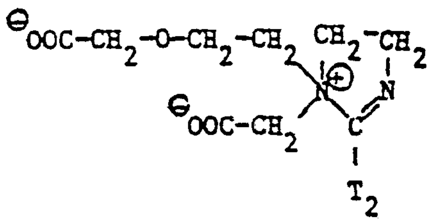

worin $T_2$ die angegebene Bedeutung hat. Als spezifische Beispiele seien die Reste der Formeln

(57) 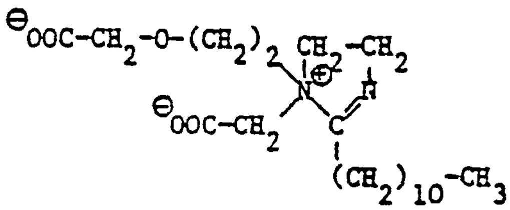 oder

(58) 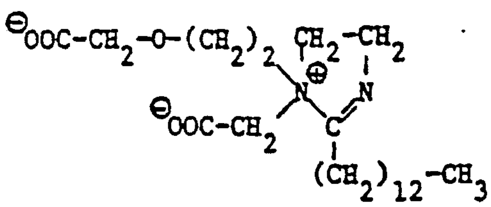

oder deren technische Gemische erwähnt.

Im Vordergrund des Interesses stehen als Bedeutungen für $X^{\ominus}$ in Formel (1) Reste von oberflächenaktiven Sarkosinaten, Sulfaten, Sulfonaten, Sulfobersteinsäurederivaten und Imidazoliniumdicarbonsäurederivaten und insbesondere die Tensidreste der Formeln (7), (15), (16), (18), (27), (28), (43), (57) und (58).

Die erfindungsgemässen copolymeren, quaternären Ammoniumsalze können nach an sich bekannten Methoden z.B. Wasser-in-Oel-Emulsionspolymerisation oder Lösungspolymerisation hergestellt werden. Ein Herstellungsverfahren besteht darin, dass man die Comonomeren der Formeln

(59) 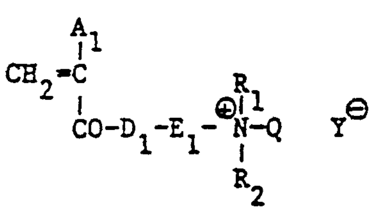

(60) 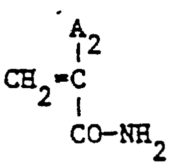

gegebenenfalls

(61) 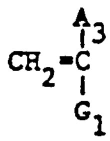

und
gegebenenfalls

(62) 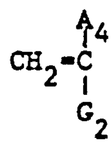 ,

worin $A_1$, $A_2$, $A_3$, $A_4$, $D_1$, $E_1$, $G_1$, $G_2$, $R_1$, $R_2$ und Q die angegebenen Bedeutungen haben und $Y^{\ominus}$ ein Halogenid-, Alkylsulfat- oder Alkylphosphonatanion mit 1 bis 4 Kohlenstoffatomen im Alkylrest bedeutet, durch Wasser-in-Oel Emulsionspolymerisation, in Gegenwart eines Wasser-in-Oel-Emulgators und gegebenenfalls eines Emulsionsstabilisators und eines Polymerisationsinitiators copolymerisiert, das Copolymerisat mit einem vorzugsweise mit Oel und Wasser mischbaren Lösungsmittel; z.B. Methanol, Aceton oder Isopropanol, ausfällt und anschliessend trocknet wobei das Herstellungsverfahren dadurch gekennzeichnet ist, dass man das erhaltene Copolymerisat mit mindestens einem anionischen Tensid oder ainem anionischen, zwitterionischen Tensid der Formel

(63) $X^{\ominus} Z^{\oplus}$,

worin $Z^{\ominus}$ ein Alkalimetall- oder ein gegebenenfalls durch ($C_1$-$C_4$)-Alkyl- oder Alkanolreste substituiertes Ammoniumkation bedeutet und $X^{\ominus}$ die angegebenen Bedeutungen hat, in wässrigem Medium bei 10 bis 60°C und einem pH-Wert von 5 bis 9 unter Austausch des Amions $Y^{\ominus}$ durch das Anion $X^{\ominus}$ umsetzt.

Arbeitet man nach der Methode der Lösungspolymerisation, so ist der Einsatz von Emulgatoren und Emulsionsstabilisatoren nicht notwendig. Als Lösungsmittel dient in der Regel Wasser.

Bei der Wasser-in-Oel Emulsionspolymerisation werden für die Oelphase hydrophobe, organische Flüssigkeiten benötigt. Zu diesem Zweck eignen sich z.B. aliphatische oder aromatische Kohlenwasserstoffe, Oele tierischer oder pflanzlicher Herkunft und die entsprechenden denaturierten Oele (z.B. hydrierte Oele, polymerisierte Oele). Zu den bevorzugten hydrophoben organischen Flüssigkeiten gehören aliphatische Kohlenwasserstoffe wie Kerosin, Paraffin, Isoparaffin und aromatische Kohlenwasserstoffe wie Benzol, Toluol und Xylol. Handelsübliche technische Gemische von vorzugsweise verzweigten Paraffinölen mit einem Siede-Bereich von 160 bis 260°C, vorzugsweise 180 bis 210°C, stehen im Vordergrund des Interesses.

Als bei der inversen Emulsionspolymerisation eingesetzte Wasser-in-Oel Emulgatoren kommen Polyoxyalkylen-, vorzugsweise Polyoxyäthylenaddukte von aliphatischen Alkoholen mit 8 bis 24 Kohlenstoffatoman wie Lauryl-, Cetyl-, Stearyl- und Oleylalkohole, von Fettsäuren mit 8 bis 24 Kohlenstoffatomen der vorstehend angegebenen Art, vorzugsweise Laurin-, Palmitin-, Stearin- und Oelsäure, von Alkylphenolen mit 8 bis 24 Kohlenstoffatomen im Alkylrest, z.B. Octyl-, Nonyl-, Dodecyl- und Dinonylphenol, und von Estern von Fettsäuren der angegebenen Art mit mehrwertigen Alkoholen, z.B. Glycerin, Pentaerythrit, Sorbit und Sorbitan in Betracht. Auch handelsübliche Gemische, d.h. Polyoxyalkylenaddukte von technischen Alkoholgemischen, Fettsäuregemischen, Alkylphenolgemischen und Estergemischen sind besonders geeignet, um als Wasser-in-Oel Emulgatoren eingesetzt zu werden. Besonders bevorzugt sind indessen Ester der Fettsäuren der angegebenen Art, bzw. Fettsäuregemische mit mehrwertigen Alkoholen der angegebenen Art, wobei Sorbitammonooleat im Vordergrund des Interesses steht.

In besonderen Fällen hat sich die Verwendung eines Emulsionsstabilisators in der Oelphase als zweckmässig erwiesen. Zu diesem Zweck eignen sich vor allem in der Oelphase lösliche Kautschuke, und zwar sowohl solche natürlicher Herkunft, z.B. Kristallgummi, als auch vorzugsweise solche synthetischer Herkunft, z.B. Polybutadien, Copolymerisate aus Styrol und Butadien und insbesondere Polyisopren, das im Vordergrund des Interesses steht.

In der Regel enthält die Oelphase etwa 2 bis 15 Gewichtsprozent Emulgator und 0 bis etwa 1, vorzugsweise 0,4 bis 0,8 Gewichtsprozent Stabilisator.

Nach dem Vermischen der Oelphase mit der wässrigen Phase, welche die Comonomere der Formeln (59), (60) und gegebenenfalls (61) und (62) enthält, wird in der Regel die Copolymerisationsreaktion durch Zugabe eines Polymerisationsinitiators in Gang gesetzt. Als Initiatoren können die üblichen Polymerisationskatalysatoren, vorzugsweise in Form ihrer wässrigen Lösungen, eingesetzt werden, z.B.

Azoverbindungen wie Azo-bis(isobutyronitril) oder Azo-bis(dimethylvaleronitril), oxydierende Mittel, vorzugsweise Peroxyde wie Wasserstoffperoxyd oder Benzoylperoxyd oder vorzugsweise Persulfate wie Armoniumpersulfat, ferner auch Chlorate oder Chromate, reduzierende Mittel, wie Sulfite, Bisulfite, Oxalsäure und Ascorbinsäure, sowie die Kombination, als sogenannte Redoxkatalysatoren, der vorstehend aufgeführten oxydierenden und reduzierenden Mittel. Im vorliegenden Fall eignet sich Natriumsulfit besonders gut als Initiator.

Die Copolymerisationsreaktion erfolgt in der Regel bei 30 bis 90°C, vorzugsweise 40 bis 70°C, und kann auch exotherm verlaufen, so dass die Polymerisationstemperatur gegebenenfalls durch Kühlen eingehalten werden muss.

Zur üblichen Aufarbeitung wird das erhaltene Copolymerisat in der Regel durch ein vorzugsweise mit Oel und Wasser mischbares Lösungsmittel gefällt, z.B. mit Methanol, Isopropanol oder Aceton, wobei die Fällung im allgemeinen durch Zugabe der Wasser-in-Oel Emulsion ins vorgelegte Lösungsmittel, vorzugsweise bei Raumtemperatur (15 bis 25°C), durchgeführt wird, worauf nach der Filtration das gefällte Polymerisat vorzugsweise bei Temperaturen von höchstens 60°C, insbesondere bei Temperaturen von etwa 30 bis 50°C, zweckmässig unter vermindertem Druck getrocknet wird.

Die an sich bekannten, so erhaltenen (Wasser-in-Oel)Copolymerisate werden nun nach an sich bekannten Methoden zu den erfindungsgemässen neuen Copolymeren, quaternären Ammoniumsalzen, die Strukturelemente der Formel (1) enthalten, umgesetzt. Zu diesem Zweck werden die erhaltenen (Wasser-in-Oel)Copolymerisate mit dem Tensid der Formel (63) wie vorstehend angegeben umgesetzt, wobei Umsetzungstemperaturen von 15 bis 40°C während 30 bis 100, insbesondere 60 bis 90 Minuten, bevorzugt sind. In der Regel wird ein Ueberschuss mindestens eines anionischen Tensids oder eines anionischen, zwitterionischen Tensids der Formel (63), bezogen auf das bei der Herstellung des Copolymerisats angewandte, comonomere, quaternäre Ammoniumsalz der Formel (59) eingesetzt. Dieser Ueberschuss beträgt etwa 4 bis 500, vorzugsweise 5 bis 120 und insbesondere 7 bis 70 Mol Tensid pro Mol des angewandten monomeren Ammoniumsalzes.

Bei ihrer Verwendung in der Kosmetikindustrie werden die erfindungsgemässen, neuen, copolymeren, quaternären Ammoniumsalze vorzugsweise als Haarkosmetika eingesetzt.

Die erfindungsgemässen kosmetischen Mittel, vorzugsweise haarkosmetische Mittel, enthalten in der Regel neben den neuen, copolymeren, quaternären Ammoniumsalzen mindestens eines der bei der Herstellung der Ammoniumsalze verwendeten anionischen oder anionischen, zwitterionischen Tenside der Formel (63) im Ueberschuss.

In ihrer bevorzugten Ausführungsform liegen die kosmetischen Mittel als wässrige Haarkosmetika vor, die 0,05 bis 1,5,vorzugsweise 0,2 bis 1,0 Gewichtsteile, berechnet als Wirksubstanz, mindestens eines polymeren Ammoniumsalzes mit Strukturelementen der Formel (1), (2), gegebenenfalls (3) und/oder (4), 5 bis 20, vorzugsweise 8 bis 15, insbesondere 9 bis 12 Gewichtsteile, berechnet als Wirksubstanz, mindestens eines anionischen Tensids oder eines anionischen, zwitterionischen Tensids der Formel (63) und gegebenenfalls kosmetische Hilfsmittel enthalten und mit entionisiertem Wasser auf insgesamt 100 Gewichtsteile verdünnt sind.

Die fakultativen, kosmetischen Hilfsmittel sind handelsübliche Mittel, wie sie in der Haarkosmetik eingesetzt werden. In Betracht kommen z.B. Tenside, die von den Tensiden der Formel (63) verschieden sind, wie Polyglycerolester von Fettsäuren, insbesondere Polygkyceroloieate oder Seifen, insbesondere Natrium-, Aluminium-, Magnesium-, Zink- oder Calci-um-Stearat, Schaumstabilisatoren' z.B. Fettsäurepolyalkanol-smide, Verdickungsmittel natürlicher oder synthetischer Herkunft wie Hydroxypropylmethylcellulose und Polyacrylsäure, Opalisierungsmittel wie Fettsäure-monoalkanol-amide oder vorzugsweise Glycerinmonostearat, ferner auch u.a. Konservierungsmittel, Parfums und Perlglanzmittel.

Erforderlichenfalls werden die haarkosmetischen Mittel auf einen pH-Wert von 5 bis 8, insbesondere von 7,0 bis 7,5 und bevorzugt von 7,0 bis 7,2 eingestellt, was zweckmässig durch einen Zusatz von u.a. einer wässrigen Lösung aus z.B. Natriumhydroxid oder Zitronensäure erreicht werden kann.

Bei der Applikation der haarkosmetischen Mittel, vorzugsweise auf menschlichem Haar, wird im Haarbehandlungsverfahren das vorstehend beschriebene, wässrige, haarkosmetische Mittel auf mit Leitungswasser angefeuchteten baar, in der Regel bei Raumtemperatur bis leicht erhöhter Temperatur, z.B. 20 bis 40°C, aufgebracht und das Haar anschliessend schamponiert und konditioniert. Das so behandelte Haar kann ebenfalls in Form von Perücken oder Toupets vorliegen.

Der wesentliche Vorteil der vorliegenden Erfindung liegt darin, dass bei der Applikation der haarkosmetischen Mittel, die copolymere, quaternäre Ammoniumsalze mit Strukturelementen der Formel (1) aus den entsprechenden Wasser-in-Oel Copolymerisaten mit hochmolekularen Anteilen und Tenside der Formel (63) enthalten, eine ausgezeichnete Trocken- und vor allem Nasskämmbarkeit des behandelten Haars erzielt wird. Insbesondere ist die Nasskämmbarkeit der mit den erfindungsgemässen haarkosmetischen Mitteln behandelten Haare der Nasskämmbarkeit von Haaren, die mit herkömmlichen kosmetischen Mitteln behandelt werden, deutlich überlegen. Letztere enthalten zwar auch Mischungen und/oder Umsetzungsprodukte aus Tensiden und Copolymerisaten, jedoch die Copolymerisate weisen eine andere Zusammensetzung auf, insbesondere fehlen ihnen die hochmolekularen Anteile.

In den nachfolgenden Herstellungsvorschriften und Beispielen angegebene Teile und Prozente sind Gewichtseinheiten.

**Herstellungsvorschriften für Wasser-in-Oel Emulsionscopolymerisate**

Vorschrift A: Die folgenden drei Lösungen werden in sauerstofffreier, inerter Stickstoffatnosphäre vorbereitet:

**Lösung 1**

(Oelphase)
In einem Doppelmantelreaktionsgefäss werden
500 Teile eines verzweigten Paraffinöls (technisches Gemisch, Molekulargewicht 171, Siedebereich 188-206°C) vorgelegt.
140 Teile einer 2,5%igen Lösung eines synthetischen Kautschuks aus Polyisopren (Emulsionsstabilisator) in Parrafinenöl der angegebenen Art und anschliessend
78 Teile Sorbitanmonooleat (Wasser-in-Oel Emulgator) werden dem vorgelegten Paraffinöl unter Rühren bei 20°C zugegeben.
Man erhält eine klare, gelbliche Lösung.

**Lösung II**

(wässrige Phase)
568 6 Teile Acrylamid (8 Mol) werden bei 20°C in
700 Teilen entionisiertem, sauerstofffreiem Wasser gelöst. In diese Lösung werden
220 Teile Natriumchlorid unter Rühren eingetragen und anschliessend äthyl-trimethylammonium-methylsulfat (2 Mol) zugegeben.
Man erhält eine klare farblose Lösung.

**Lösung III**

(Initiatorlösung)
0,66 Teile Natriumsulfit werden in
40 Teilen entionisiertem, sauerstofffreiem Wasser gelöst.

**Copolymerisationsreaktion**

Unter intensivem Rühren (3000 U/min) wird innerhalb von 10 Minuten in inerter Stickstoffatmosphäre die Lösung II in die vorgelegte Lösung I bei 20°C zugegeben. Man erhält eine homogene, weisse Emulsion, die bei 20°C weitergerührt wird, bis die Viskosität einer Emulsionsprobe 14000 m Pa.s (Brookfield Viskosimeter LV, Spindel 3, 6 U/min, 25°C) beträgt, was im allgemeinen 10 Minuten in Anspruch nimmt. Hierauf wird das Reaktionsgemisch unter Rühren bei 300 U/min innerhalb von 30 Minuten auf 40°C geheizt. Mittels einer Dosierpumpe wird nun die Lösung III innerhalb von 150 Minuten in das Reaktionsgemisch zugegeben, wobei die Temperatur durch Kühlen auf 40 bis 41°C gehalten wird. Nach Beendigung der Initiatorlösung-Zugabe wird das Reaktionsgemisch bei 40°C und 300 U/min weiter gerührt, bis die Viskosität einer Emulsionsprobe auf 7600 mPa.s (Brookfield Viskosimeter LV, Spindel 1, 60 U/min, 25°C) absinkt, was im allgemeinen eine Stunde in Anspruch nimmt.

**Aufarbeitung**

Die erhaltene Emulsion des Copolymerisates wird unter Rühren in 24000 Teile Aceton bei 20°C gegeben und das Copolymerisat ausgefällt. Das ausgefällte Copolymerisat wird abfiltriert und während 2 Tagen unter vermindertem Druck bei 40°C getrocknet. Man erhält 1100 Teile eines Copolymerisates, das in beliebiger Reihenfolge 80 Mol% Strukturelemente der Formel

(64) 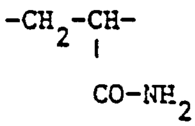

und
20 Mol% Strukturelemente der Formel

(65) 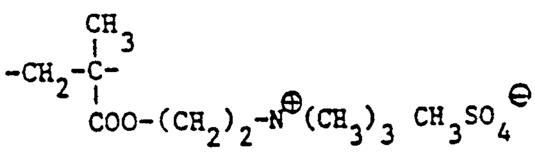

enthält, wobei das Molekulargewicht von 37% des Copolymerisates zwischen $10^7$ und $10^9$ liegt.

**Vorschrift B**

Man verfährt wie in Vorschrift A angegeben, setzt jedoch in der wässrigen Phase (Lösung II) 1108 Teile einer 40%igen, wässrigen Lösung aus Methacryloyloxypropyl-trimethylammonium-chlorid (2 Mol) ein.
Man erhält 1000 Teile eines Copolymerisates, das in beliebiger Reihenfolge
80 Mol% Strukturelemente der in Vorschrift A angegebenen Formel (64) und
20 Mol% Strukturelemente der Formel

(66) 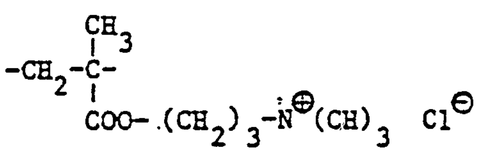

enthält, wobei das Molekulargewicht von 27% des Copolymerisats zwischen $10^7$ und $10^9$ liegt.

**Vorschrift C**

Man verfährt wie in Vorschrift A angegeben, setzt jedoch in der wässrigen Phase (Lösung II) 1622 Teile einer 40%igen, wässrigen Lösung aus Methacryloyl-amidopropyl-diäthylmethylammonium-methylsulfat (2 Mol) ein.

Man erhält 648,8 Teile eines Copolymerisats, das in beliebiger Reihenfolge
80 Mol% der in Vorschrift A angegebenen Formel (64) und
20 Mol% Strukturelemente der Formel

(67) 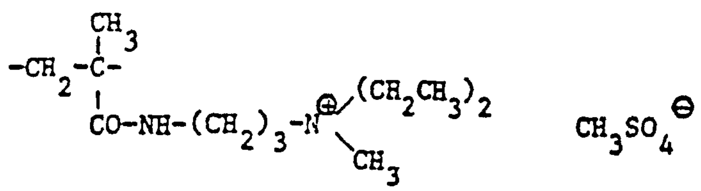

enthält, wobei das Molekulargewicht von 7% des Copolymerisats zwischen $10^7$ und $10^9$ liegt.

**Vorschrift D**

Man verfährt wie in Vorschrift A angegeben, setzt jedoch in der wässrigen Phase (Lösung II) 1471,5 Teile einer 40%igen, wässrigen Lösung aus Methacryloyl-oxypropyl-dimethyl-n-propyl-ammoniumbromid (2 Mol) ein.

Man erhält 1000 Teile eines Copolymerisates, das in beliebiger Reihenfolge
80 Mol% Strukturelemente der in Vorschrift A angegebenen Formel (64) und
20 Mol% Strukturelemente der Formel

(68) 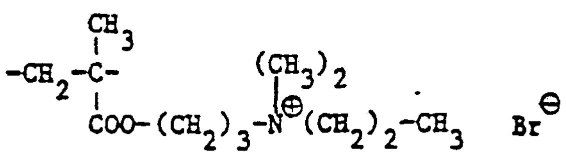

enthält, wobei das Molekulargewicht von 22% des Copolymerisats zwischen $10^7$ und $10^9$ liegt.

**Vorschrift E**

Man verfährt wie in Vorschrift A angegeben, setzt jedoch die nachfolgenden Lösungen I, II und III ein und verwendet bei der Aufarbeitung 40000 Teile Aceton.

**Lösung I**

825 Teile des in Vorschrift A angegebenen Paraffinöls,
269 Teile der in Vorschrift A angegebenen 2,5%igen Kautschuklösung
255 Teile Sorbitanmonooleat.

**Lösung II**

wässrige Lösung aus
681 6 Teile Acrylamid (9,6 Mol),
34,6 Teile Acrylsäure (0,48 Mol),
50,3 Teile Dimethylamino-methacrylsäure-Äthylester (0,32 Mol) und
220 Teile Natriumchlorid in 700 Teilen Wasser.

Wässrige Lösung aus 1586,5 Teilen Methacryloyl-oxyäthyl-trimethylammonium-methylsulfat (5,6 Mol) in 3 100 Teilen Wasser.

**Lösung III**

1,07 Teile Natriumsulfit in
66,8 Teilen Wasser.

Man erhält 2100 Teile eines Copolymerisats, das in beliebiger Reihenfolge
60 Mol% Strukturelemente der in Vorschrift A angegebenen Formel (64)
35 Mol% Strukturelemente der in Vorschrift A angegebenen Formel (65),
3 Mol% Strukturelemente der Formel

(69) 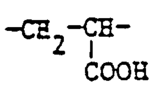

und
2 Mol% Strukturelemente der Formel

(70) 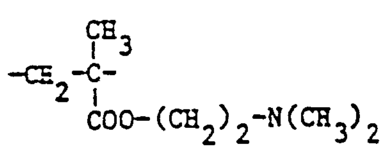

enthält, wobei das Molekulargewicht von 12% des Copolymerisats zwischen $10^7$ und $10^9$ liegt.

**Vorschrift F**

Man verfährt wie in Vorschrift A angegeben, setzt jedoch die nachfolgenden Lösungen I, II und III ein und vervendet bei der Aufarbeitung 50000 Teile Aceton.

**Lösung I ***

1628 Teile des in Vorschrift A angegebenen Paraffinöls und
191 Teile Sorbitan-monooleat
(* enthält keine Kautschuklösung im Gegensatz zu Vorschrift A).

**Lösung II**

Lösung aus
1516,6 Teilen Acrylamid** (21,36 Mol),
679,9 Teile Methacryloyl-oxyäthyl-trimethylammonium-methylsulfat (2,40 Mol),
37,7 Teilen Dimethylamino-methacrylsäure-Äthylester** (0,24 Mol) und
600 Teilen Natriumchlorid** in
2550 Teilen Wasser
(** diese Komponenten werden entgegen Vorschrift A nicht separat vorgelegt).

**Lösung III**

1,6 Teile Natriumsulfit,
720 Teilen Wasser.
Man erhält 1890 Teile eines Copolymerisats, das in beliebiger Reihenfolge
90 Mol% Strukturelemente der in Vorschrift A angegebenen Formel (64)
9 Mol% Strukturelemente der in Vorschrift A angegebenen Formel (65)
und
1 Mol% Strukturelemente der in Vorschrift E angegebenen Formel (70)
enthält, wobei das Molekulargewicht von 23% des Copolymerisats zwischen $10^7$ und $10^9$ liegt.

**Herstellungsvorschrift für Lösungscopolymerisat**

Vorschrift G: Die folgenden zwei Lösungen werden in sauerstofffreier inerter Stickstoffatmosphäre vorbereitet:

**Lösung I**

(Monomerlösung)
In einem Doppelmantelgefäss werden
71,1 Teile Acrylamid (1 Mol) und
141,7 Teile einer 50%igen, wässrigen Lösung aus Methacryloyloxy-Äthyl-trimethylammonium-methylsulfat (0,25 Mol) bei 20°C in
228 Teilen entionisiertem, sauerstofffreiem Wasser gelöst.
Man erhält eine klare farblose Lösung.

**Lösung II**

(Initiatorlösung)
0,2 Teile Ammoniumperoxodisulfat werden in
150 Teilen deionisiertem sauerstofffreiem Wasser gelöst.

**Copolymerisationsreaktion**

Unter Rühren wird innerhalb 1 Minute in inerter Stickstoffatmosphäre die Hälfte der Lösung 11 in die vorgelegte Lösung I bei 35°C zugegeben. Nach 6 Stunden wird die Reaktionslösung auf 50° C ervärmt und die zweite Hälfte von Lösung II zugegeben. Es wird solange gerührt, bis nach 2 bis 3 Stunden eine hochviskose Lösung entstanden ist. Ohne Rühren wird das Reaktionsgemisch stehen gelassen. Nach 24 Stunden lässt man das entstandene farblose Gel abkühlen.

**Aufarbeitung**

Das Gel wird zerkleinert und in 1350 Teilen deionisiertem Wasser gelöst. Die hochviskose Lösung wird dann in 18000 Teilen Aceton in dünnem Strang bei 20°C eingepresst und das Copolymerisat ausgefällt. Das Copolymerisat wird dann abfiltriert und nochmals in 1800 Teilen Aceton geknetet, bis es hart und spröde wird. Das Copolymerisat wird wieder abfiltriert und während 2 Tagen unter vermindertem Druck bei 40°C getrocknet. Man erhält 110 Teile eines Copolymerisats, das in beliebiger Folge 80 Mol% Strukturelemente der Formel

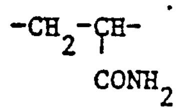

und 20 Mol%

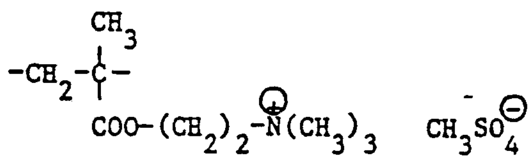

enthält, wobei das Molekulargewicht von 12 % des Copolymerisats zwischen $10^7$ und $10^9$ liegt.

**Beispiel 1**

100 Teile einer wässrigen Lösung, die 1,71 Teile des Copolymerisats gemäss Vorschrift A (3 m-Aequivalente bezogen auf den Quatgehalt des Copolymerisats), enthalten, werden in eine vorgelegte Lösung aus 24,9 Teilen des Tensids der Formel

(71) $CH_3-(CH_2)_{11}-O-SO_3H.N(CH_2-CH_2OH)_3$ (70 mMol oder 23,3 Mol pro Mol des in Vorschrift A angewandten, comonomeren, quaternären Ammoniumsalzes) in 150 Teilen Wasser innerhalb von 80 Minuten bei 35°C zugegeben, wobei gleichzeitig die Umsetzung der quaternären Strukturelemente des Copolymerisates mit dem eingesetzten Tensid unter Ionenaustausch erfolgt. Man erhält 250 Teile einer wässrigen, schwach opaleszierenden, viskosen Lösung, die 9 5% Tensid der vorstehend angegebenen Formel (71) und 0,9% eines Copolymerisats enthält, das in beliebiger Reihenfolge

80 Mol% Strukturelemente der in Vorschrift A angegebenen Formel (64)

und

20 Mol% Strukturelemente der Formel

(72) 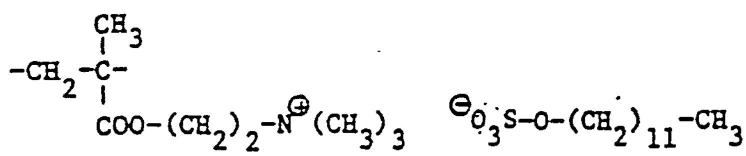

aufweist.

**Beispiel 2**

Man verfährt wie in Beispiel 1 angegeben, setzt jedoch 7,7 Teile des Tensids der Formel

(73) $CH_3(CH_2)_{11}-O-(CH_2-CH_2-O)_2-SO_3Na$

(20 mMol oder 6,67 Mol pro Mol der in Vorschrift A angewandten, comonomeren, quaternären Ammoniumsalze) ein.

Man erhält 250 Teile einer wässrigen, schwach opaleszierenden Lösung, die 2,6% Tensid der vorstehenden angegebenen Formel (73) und

1,0% eines Copolymerisats enthält, das in beliebiger Reihenfolge

80 Mol% Strukturelemente der in Dorschrift A angegebenen Formel (64)

und
20 Mol% Strukturelemente der Formel

(74) 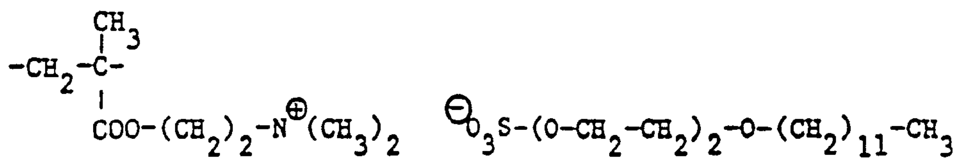

aufweist.

**Beispiel 3**

Man verfährt wie in Beispiel 1 angegeben, setzt jedoch 29,3 Teile des Tensids der Formel

(75) 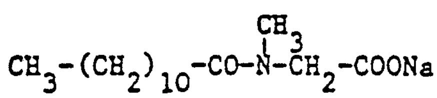

(100 mMol oder 33,3 Mol pro Mol des in Vorschrift A angewandten, comonomeren, quaternären Ammoniumsalzes) ein. Man erhält 250 Teile einer wässrigen, schwach opaleszierenden viskosen Lösung, die 11,3% des Tensides der vorstehend angegebenen Formel (75) und 0,9% eines Copolymerisates enthält, das in beliebiger Reihenfolge
80 Mol% Strukturelemente der in Vorschrift A angegebenen Formel (64)
und
20 Mol% Strukturelemeute der Formel

(76) 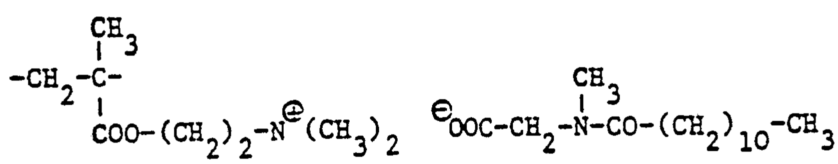

aufweist.

**Beispiel 4**

Man verfährt wie in Beispiel 1 angegeben setzt jedoch 25,7 Teile des Tensides der Formel

(77) 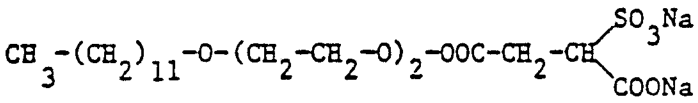

(50 mMol oder 16,7 Mol pro des in Vorschrift A angewandten, comonomeren, quaternären Ammoniumsalzes) ein.

Man erhält 250 Teile einer wässrigen, schwach opaleszierenden Lösung, die 9,7% des Tensides der vorstehend angegebenen Formel (77) und 1,1% eines Copolymerisats enthält, das in beliebiger

80 Mol% Strukturelemente der in Vorschrift A engegebenen Formel (64)

und

20 Mol% Strukturelemente der Formel

(78) 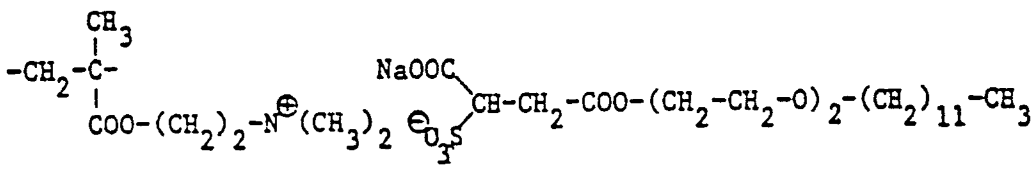

aufweist.

**Beispiel 5**

Man verfährt wie in Beispiel 1 angegeben, setzt jedoch 25,2 Teile des Tensides der Formel

(79) 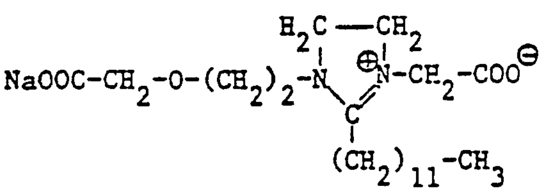

(60 mMol oder 20 Mol pro Mol des in Vorschrift A angewandten, comonomeren, quaternären Ammoniumsalzes) ein.

Man erhält 250 Teile einer wässrigen, schwach opaleszierenden Lösung, die 9,6% Tensid der vorstehend angegebenen Formel (79) und 1,1% eines Copolymerisats enthält, das in beliebiger Reihenfolge

80 Mol% Strukturelemente der in Vorschrift A angegebenen Formel (64)

und

20 Mol% Strukturelemente der Formel

(80) 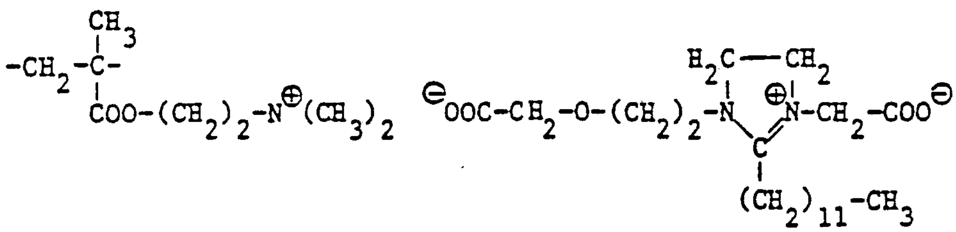

aufweist.

**Beispiel 6**

Man verfährt wie in Beispiel 1 angegeben, setzt jedoch 24,4 Teile des Tensides der Formel

(81) 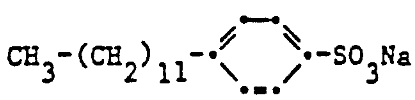

(70 mMol oder 22,3 Mol pro Mol des in Vorschrift A angewandten, comonomeren, quaternären Ammoniumsalzes) ein.
Man erhält 250 Teile einer wässrigen, schwach opaleszierenden Lösung, die 9,3% Tensid der vorstehend angegebenen Formel (81) und 0,85% eines Copolymerisats enthält, das in beliebiger Reihenfolge
80 Mol% Strukturelemente der in Vorschrift A angegebenen Formel (64)
und
20 Mol% Strukturelemente der Formel

(82) 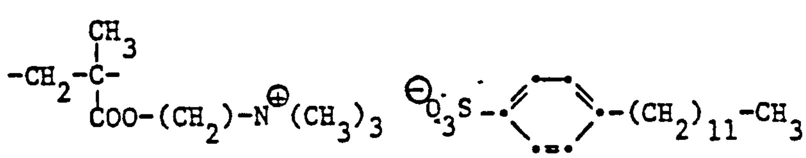

aufweist.

**Beispiel 7**

100 Teile einer wässrigen Lösung, die 2,02 Teile des Copolymerisats gemäss Vorschrift B (4 m Aequivalente bezogen auf den Quatgehalt des Copolymerisats) enthält, werden in eine vorgelegte Lösung aus 105 Teilen des Tensids der in Beispiel 5 angegebenen Formel (79) (250 mMol oder 62,5 Mol pro Mcl des in Vorschrift B

angewandten, comonomeren, quaternären Ammoniumsalzes) in 900 Teilen Wasser innerhalb von 1 Stunde bei 25°C zugegeben, wobei gleichzeitig die Umsetzung der quaternären Strukturelemente des Copolymerisats mit dem eingesetzten Tensid unter Ionenaustausch erfolgt. Man erhält 1000 Teile einer wässrigen, schwach opaleszierenden, viskosen Lösung, die 10,4% Tensid der vorstehend angegebenen Formel (79) und 0,31% eines Copolymerisats enthält, das in beliebiger Reihenfolge
80 Mol% Strukturelemente der in Vorschrift A angegebenen Formel (64)
und
20 Mol% Strukturelemeute der in Beispiel 5 angegebenen Formel (80) aufweist.

**Beispiel 8**

Man verfährt wie in Beispiel 7 angegeben, setzt jedoch 104 Teile des Tensids der in Beispiel 1 angegebenen Formel (71) (250 mMol oder 62,5 Mol pro Mol des in Vorschrift B angewandten, comonomeren, quaternären Ammoniumsalzes) ein.
Man erhält 1000 Teile einer wässrigen, schwach opaleszierenden Lösung, die 10,3% des Tensids der vorstehend angegebenen Formel (71) und 0,3% eines Copolymerisats enthält, das in beliebiger Reihenfolge 80 Mol% Strukturelemente der in Vorschrift A angegebenen Formel (64)
und
20 Mol% Strukturelemente der in Beispiel 5 angegebenen Formel (80) aufweist.

**Beispiel 9**

10 Teile einer wässrigen Lösung, die 0,168 Teile des Copolymerisats gemäss Vorschrift E (0,4 m Aequivalente bezogen auf den Quatgehalt des Copolymerisats), enthält, werden in eine vorgelegte Lösung aus 10,5 Teilen des Tensids der in Beispiel 1 angegebenen Formel (71)
(5,71 mMol oder 14,3 Mol pro Mol des in Vorschrift E angewandten, comonomeren Ammoniumsalzes) in 90 Teilen Wasser innerhalb von 90 Minuten bei 35°C zugegeben, wobei gleichzeitig die Umsetzung der quaternären Strukturelemente des Copolymerisates mit dem eingesetzten Tensid unter Ionenaustausch erfolgt. Man erhält 100 Teile einer wässrigen, schwach opaleszierenden, viskosen Lösung, die 9,8% Tensid der vorstehend angegebenen Formel (71) und 0,38% eines Copolymerisates enthält, das in beliebiger Reihenfolge
60 Mol% Strukturelemente der in Vorschrift A angegebenen Formel (64),
3 Mol% der in Vorschrift E angegebenen Formel (69),
2 Mol% der in Vorschrift E angegebenen Formel (70) und
35 Mol% Strukturelemente der in Beispiel 5 angegebenen Formel (80) aufweist.

**Beispiel 10**

35 Teile einer wässrigen Lösung, die 0,65 Teile des Copolymerisats gemäss Vorschrift E (0,7 m Aequivalente bezogen auf den Quatgehalt des Copolymerisates) enthält, werden in eine vorgelegte Lösung aus 13,7 Teilen des Tensides der Formel

(83) $CH_3(CH_2)_{10}-CO-\overset{CH_3}{\overset{|}{N}}-(CH_2)_2SO_3Na$

(40 mMol oder 57,1 Mol pro Mol des in Vorschrift E angewandten, cononomeren quaternären Ammoniumsalzes) in 65 Teilen Wasser inuerhalb von 75 Minuten bei 30°C zugegeben, wobei gleichzeitig die Umsetzung der quaternären Strukturelemente des Copolymerisates mit dem eingesetzten Tensid unter Ionenaustausch erfolgt.
Man erhält 100 Teile einer wässrigen, schwach opaleszierenden, viskosen Lösung, die 13,5% Tensid der vorstehend angegebenen Formel (83) und 0,75% eines Copolymerisats enthält, das in beliebiger Reihenfolge
90 Mol% Strukturelemente der in Dorschrift A angegebenen Formel (64)
1 Mol% Strukturelemente der in Vorschrift A angegebenen Formel (65)
und

9 Mol% Strukturelemente der Formel

(84) 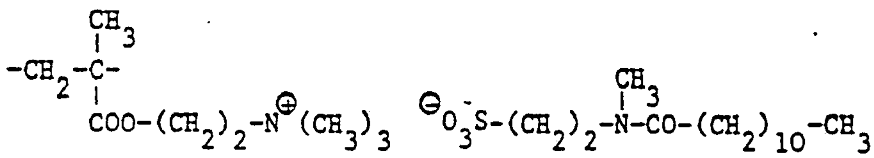

aufweist.

**Beispiel 11**

50 Teile einer wässrigen Lösung, die 0,58 Teile des Copolymerisats gemäss Vorschrift D (1 m Aequivalent bezogen auf den Quatgehalt des Copolymerisats) enthält, werden in eine vorgelegte Lösung aus 11,6 Teilen des Tensides der in Beispiel 2 angegebenen Formel (73) (30 mMol, d.h. 30 Mo, pro Mol des in Vorschrift D angewandten, comonomeren quaternären Ammoniumsalzes) in 50 Teilen Wasser innerhalb von 50 Minuten bei 45°C zugegeben, wobei gleichzeitig die Umsetzung der quaternärem Strukturelemente des Copolymerisates mit dem eingesetzten Tensid unter Ionenaustausch erfolgt.

Man erhält 100 Teile einer wässrigen, schwach opaleszierenden viskosen Lösung, die 11,2% Tensid der vorstehend angegebenen Formel (73) und 0,87% eines Copolymerisates enthält, das in beliebiger Reihenfolge
80 Mol% Strukturelemente der in Vorschrift A angegebenen Formel (64)
und
20 Mol% Strukturelemente der Formel

(85) 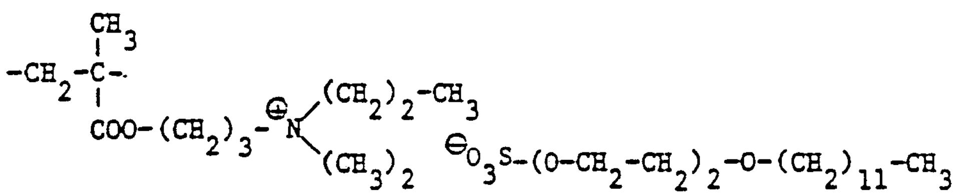

aufweist.

**Beispiel 12**

Die wässrige Lösung gemäss Beispiel 1 wird durch Zugabe einer 10%igen, wässrigen Zitronensäurelösung auf den pH-Wert vom 7,1 gestellt und auf eine mit Leitungswasser augefeuchtete Perücke aus ungebleichtem, ungefärbtem, braunem Menschenhaar europäischer Herkunft bei 30°C in dreimaliger Applikation im sogenannten Halbseitentest aufgebracht, worauf das Perückenhaar bei dieser Temperatur schamponiert und konditioniert wird. Im Halbseitentest wird nun die eine Hälfte der Perücke mit der vorstehend genannten Lösung schamponiert und konditioniert, während die andere Hälfte der Perücke mit einer Lösung unter gleichen Bedingungen schamponiert wird, die kein erfindungsgemässes Copolymerisat, sondern nur Tensid

enthält. In dieser sogenannten Leerformulierung wird die Menge an Copolymerisat durch die entsprechende Menge Tensid ersetzt, so dass z.B. die Leerformulierung als Vergleich zum Beispiel 1 10,4% Tensid der in Beispiel 1 angegebenen Formel (71) enthält, wobei der pH-Wert der Leerformulierung ebenfalls mit der wässrigen, 10%igen Zitronensäurelösumg auf den pH-Wert von 7,1 gestellt wird. Nach jeder Applikation wird die Nass- und Trockenkämmbarkeit der erfindungsgemäss behandelten Perückenhälfte im Vergleich mit der mit der Leerformulierung behandelten Perückenhälfte mit Hilfe der nachfolgenden Notenskala beurteilt:

+3 viel besser als die Leerformulierung
+2 besser als die Leerformulierung
+1 etwas besser als die Leerformulierung
0 kein Unterschied zur Leerformulierung
-1 etwas schlechter als die Leerformulierung
-2 schlechter als die Leerformulierumg
-3 viel schlechter als die Leerformulierung.

Die unter Verwendung der Lösung gemäss Beispiel 1 erzielten Kämmbarkeitsnoten sind in der nachfolgenden Tabelle 1 zusammengefasst.

**Tabelle I**

| | nach der 1. Applikation | nach der 2. Applikation | nach der 3. Applikation |
|---|---|---|---|
| Nasskämmbarkeit | +2 | +3 | +3 |
| Trockenkämmbarkeit | +2 | +(2−3) | +3 |

Aehnliche Ergebnisse werden mit der wässrigen Lösung gemäss Beispiel 3 und den entsprechenden Leerformulierungen erzielt.

**Beispiel 13**

Man verfährt wie im Beispiel 12 angegeben, setzt jedoch die wässrige Lösung gemäss Beispiel 4 und die entsprechende Leerformulierung ein.

Die erzielten Kämmbarkeitsnoten sind in der nachfolgenden Tabelle II zusammengefasst.

**Tabelle II**

| | nach der 1. Applikation | nach der 2. Applikation | nach der 3. Applikation |
|---|---|---|---|
| Nasskämmbarkeit | +(1−2) | +(1−2) | +(1−2) |
| Trockenkämmbarkeit | +1 | +(1−2) | +(1−2) |

**Beispiel 14**

Man verfährt wie im Beispiel 12 angegeben, setzt jedoch die wässrige Lösung gemäss Beispiel 5 und die entsprechende Leerformulierung ein.

Die erzielten Kämmbarkeitsnoten sind in der nachfolgenden Tabelle III angegeben.

**Tabelle III**

| | nach der 1. Applikation | nach der 2. Applikation | nach der 3. Applikation |
|---|---|---|---|
| Nasskämmbarkeit | +(1−2) | +2 | +2 |
| Trockenkämmbarkeit | +(0−1) | +(0−1) | +1 |

Aehnliche Ergebnisse werdeu mit den wässrigen Lösungen gemäss Beispielen 6 und 7 und den entsprechenden Leerformulierungen erzielt.

**Beispiel 15**

Man verfährt wie in Beispiel 12 engegeben, setzt jedoch die wässrige Lösung gemäss Beispiel 8 und die entsprechende Leerformulierung ein.
Die erzielten Kämmbarkeitsnoten sind in der nachfolgenden Tabelle IV angegeben.

**Tabelle IV**

| | nach der 1. Applikation | nach der 2. Applikation | nach der 3. Applikation |
|---|---|---|---|
| Nasskämmbarkeit | +(0−1) | +1 | +1 |
| Trockenkämmbarkeit | +1 | +(0−1) | +1 |

**Beispiel 16**

Man verfährt wie im Beispiel 12 angegeben, setzt jedoch die wässrige Lösung gemäss Beispiel 2 und die entsprechende Leerformulierung ein.
Die erzielten Kämmbarkeitsnoten sind in der nachfolgenden Tabelle V zusammengefasst.

**Tabelle V**

| | nach der 1. Applikation | nach der 2. Applikation | nach der 3. Applikation |
|---|---|---|---|
| Nasskämmbarkeit | +1 | +2 | +1−2) |
| Trockenkämmbarkeit | +1 | +2 | +2 |

Aehnliche Ergebnisse werden mit der wässrigen Lösung gemäss Beispiel 8 und der eutsprechenden Leerformulierung erzielt.

**Beispiel 17**

Man verfährt wie im Beispiel 12 augegeben, setzt jedoch die wässrige Lösung gemäss Beispiel 9 und die entsprechende Leerformulierung ein.

Die erzielten Nasskämmbarkeitsnoten betragen nach der 1. Applikation +I und nach der 2. und 3. Applikation jeweils +(1-2)

**Beispiel 18**

Man verfährt wie im Beispiel 12 angegeben, setzt jedoch die wässrige Lösung gemäss Beispiel 10 und die entsprechende Leerformulierung ein.

Die erzielten Kämmbarkeitsnoten sind in der nachfolgenden Tabelle VI zusammengefasst.

**Tabelle VI**

| | nach der 1. Applikation | nach der 2. Applikation | nach der 3. Applikation |
|---|---|---|---|
| Nasskämmbarkeit | +2 | +2 | +2 |
| Trockenkämmbarkeit | +1 | +1 | +1 |

Aehnliche Ergebnisse werden mit der wässrigen Lösung gemäss Beispiel 11 und der entsprechenden Leerformulierung erzielt.

**Beispiel 19**

100 Teile eines Copolymerisats, das zu 74 Mol-% aus Strukturelementen der Formel (64) und zu 26 Mol-% aus Elementen der Formel (65) besteht, werden pulverisiert und mit einem Gemisch von 400 Teilen Methanol und 100 Teilen Wasser bei Raumtemperatur 30 Minuten lang gerührt. Weitere 500 Teile Methanol werden zugesetzt und das Gemisch noch 5 Minuten gerührt und dann eine Stunde stehen gelassen. Der gelige Brei wird dann in einer Drucknutsche unter einem Druck von 3 bar filtriert. Die gelige Masse wird enschliessend mit 100 Teilen Methanol aufgeschlämmt und wieder unter Druck abfiltriert. Nach dreimaliger Behandlung mit Methanol wird das Produkt einen Tag im Vakuum bei 40°C getrocknet.

Die Molekulargewichtsverteilung im Ausgangspolymer (A) bzw. in dem nach obiger Vorschrift behandelten Polymer (B) ist gemäss den mittels Gelpermeationschronatographie erhaltenen Werten wie folgt:

| Polymer | $M \geq 10^7$ | $M = 10^5 - 10^7$ | $M < 10^5$ |
|---|---|---|---|
| A | 20 % | 29 % | 51 % |
| A | 39 % | 48 % | 13 % |

Mit den beiden Polymeren A und B werden Formulierungen zubereitet, indem man nach den Angaben des Beispiels 1 jeweils 1,4 Teile eines der beiden Polymeren, gelöst in 80 Teilen Wasser, zu einer Lösung von 20 Teilen der Verbindung der Formel $CH_3(CH_2)_{11}OSO_3H.N(CH_2CH_2OH)_3$ in 120 Teilen Wasser gibt.

Werden diese Formulierungen, wie im Beispiel 12 beschrieben, im Halbseitentest auf Perücken geprüft, so zeigen beide gegenüber der Leerformulierung eine bessere Nasskämmbarkeit.

Bei der Formulierung mit dem gereinigten Polymer B ist diese Wirkung jedoch stärker ausgeprägt.

| | Nasskämmbarkeit | | |
|---|---|---|---|
| | nach der 1. Applikation | nach der 2. Applikation | nach der 3. Applikation |
| Formulierung mit Polymer A | +1–2 | +2 | +2 |
| Formulierung mit Polymer B | +2 | +3 | +3 |

**Patentansprüche**

1. In wässrigen Tensidsystemen lösliche oder mikroemulgierbare, copolymere, quaternäre Ammoniumsalze, dadurch gekennzeichnet, dass sie eine Molekulargewichtsverteilung von $10^4$ bis $10^9$ auf weisen, wobei das Molekulargewicht von mindestens 5 Gewichtsprozent des Copolymers $10^7$ bis $10^9$ beträgt, und in beliebiger Reihenfolge

durchschnittlich 5 bis 80 Mol % wiederkehrende Strukturelemente der Formel

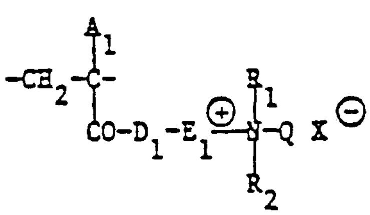

,

durchschnittlich 10 bis 95 Mol % wiederkehrende Strukturelemente der Formel

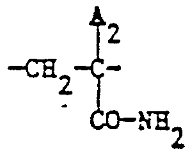

und
0 bis durchschnittlich 10 Mol % Strukturelemente der Formeln

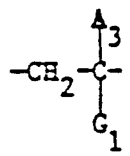

und gegebenenfalls

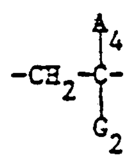.

enthalten, woin $A_1$, $A_2$, $A_3$ und $A_4$ je Wasserstoff oder Methyl, $G_1$ und $G_2$ voneinander verschieden sind und je -CN, -COOH oder -CO-$D_2$-$E_2$

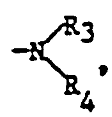,

$D_1$ und $D_2$ je Sauerstoff oder -NH-, $E_1$ und $E_2$ je Alkylen mit 1 bis 4 Kohlenstoffatomen, das gegebenenfalls durch Hydroxyl substituiert ist, $R_1$, $R_2$, $R_3$ und $R_4$ je Methyl oder Aethyl, Q Alkyl, Hydroxyalkyl mit 1 bis 4 Kohlenstoffatomen oder oder Benzyl und $X^{\ominus}$ den Rest mindestens eines anionischen Tensids oder eines anionischen, zwitterionischen Tensids bedeuten.

2. Ammoniumsalze nach Anspruch I, dadurch gekennzeichnet, dass sie durch Wasser-in-Oel Emulsionspolymerisation oder Lösungspolymerisation eines quaternären Ammoniumsalzes der Acrylsäurereihe

und mindestens eines weiteren Comonomeren auf Acrylbasis, Isolierung des Copolymerisats und Umsetzung mit einem anionischen oder anionischen, zwitterionischen Tensides unter Ionenaustausch erhältlich sind.

3. Ammoniumsalze nach einem der Ansprüche 1 oder 2, dadurch gekennzeichnet, dass das Molekulargewicht von 5 bis 60 Gewichtsprozent des Copolymerisats $10^7$ bis $10^9$ beträgt.

4. Ammoniunsalze nach einem der Amsprüche 1 bis 3, dadurch gekennzeichnet, dass $X^{\ominus}$ in der angegebenen Formel des Anspruchs 1 den Rest eines oberflächenaktiven Sarkosinates Sulfats (Sulfatäthers), Sulfonates oder Sulfobernsteinsüurederivates, eines fluorierten Tensids oder eines Phosphatrensides bedeutet.

5. Ammoniumsalze nach einem der Ansprüche 1 bis 3, dadurch gekennzeichnet, dass $X^{\ominus}$ in der angegebenen Formel des Anspruchs 1 den Rest eines zvitrerionischen oberflächenaktiven N-alkyl-α-iminodiproprionars oder eines in 2-Stellung alkylsubstituierten Imidazoliniumdicarbonsäurederivats bedeutet.

6. Verfahren zur Herstellung des Ammoniumsalzes gemäss einem der Ansprüche 1 bis 5, wobei man die Comonomeren der Formel

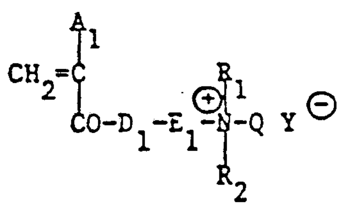

,

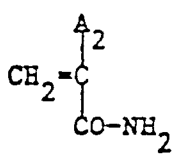

,

gegebenenfalls

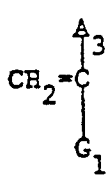

und gegebenenfalls

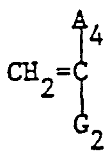

,

worin $A_1$, $A_2$, $A_3$, $A_4$, $D_1$, $E_1$, $G_1$, $G_2$, $R_1$, $R_2$ und Q die in Anspruch 1 angegebenen Bedeutungen haben und $Y^{\ominus}$ ein Halogenid-, Alkylsulfatoder Alkylphosphonatanion mit 1 bis 4 Kohlensroffatomen im Alkylrest bedeuten, durch Wasser-in-Oel Emulsionspolymerisation in Gegenwart eines Wasser-in-Oel Emulgators und gegebenenfalls eines Emulsionsstabilisators oder durch Lösungspolymerisation, jeweils in Gegenwart eines Polymerisationsinitiators, copolymerisiert, das Copolymerisat mit einem wasserlöslichen Lösungsmittel ausfällt und anschliessend trocknet, dadurch gekennzeichnet, dass nan das so erhaltene Copolymerisat mit mindestens einem anionischen Tensid oder einem anionischen, zwitterionischen Tensid der Formel

$X^{\ominus} Z^{\ominus}$,

worin $Z^{\ominus}$ ein Alkalimetall- oder ein gegebenenfalls durch ($C_1$-$C_4$)-Alkyl- oder Alkanolreste substituiertes Ammoniumkation bedeurer und $X^{\ominus}$ die in Anspruch 1 angegebene Bedeutung hat, in wässrigem Medium bei 10 bis 60°C und einem pH-Wert von 5 bis 9 unrer Austausch des Anions $Y^{\ominus}$ durch das Anion $X^{\ominus}$ umsetzt.

7. Verfahren nach Anspruch 6, dadurch gekennzeichnet, dass man die Umsetzung unter lonenaustausch bei 15 bis 40°C während 30 bis 100 Minuten durchführt.

8. Verfahren nach einem der Ansprüche 6 oder 7, dadurch gekennzeichnet, dass man einen Ueberschuss mindestens eines anionischen Tensids oder eines anionischen, zwitterionischen Tensids der in Anspruch 6 angegebenen Formel, bezogen auf das bei der Herstellung des Copolymerisats verwendete monomere, quaternäre Ammoniumsalz, einsetzt.

9. Verfahren nach Anspruch 8, dadurch gekennzeichnet, dass man 4 bis 500 Mol Tensid pro Mol monomeres Ammoniumsalz einsetzt.

10. Verwendung der polymeren Ammoniumsalze gemäss einem der Ansprüche 1 bis 5 in kosmetischen Mitteln.

11. Verwendung nach Anspruch 10, dadurch gekennzeichnet, dass die polymeren Ammoniumsülze in haarkosmetischen Mitteln eingesetzt werden.

12. Kosmetische Mittel, dadurch gekennzeichnet, dass sie mindestens ein polymeres Ammoniumsalz gemäss einem der Ansprüche 1 bis 5 enthalten.

13. Kosmetische Mittel nach Anspruch 12, dadurch gekennzeichnet, dass sie mindestens eines der bei der Hersrellung des Ammoniumsalzes verwendeten, anionischen, oder anionischen, zwitterionischen Tenside der in Anspruch 6 angegebenen Formel in Ueberschuss enthalten.

14. Kosmetische Mittel nach Anspruch 13, dadurch gekennzeichnet, dass sie Haarkosmetika sind.

15. Haarkosmetische Mittel nach Anspruch 14, dadurch gekennzeichnet, dass sie 0,05 bis 1,5 Gewichtsteile, berechnet als Wirksubstanz, mindestens eines polymeren Ammoniunsalzes gemäss einem der Ansprüche 1 bis 5, 5 bis 20 Gewichtsteile, berechnet als Wirksubstanz, mindestens eines anionischen Tensids oder eines anionischen, zwitterionischen Tensids und gegebenenfalls kosmetische Hilfsmittel enthalten und mit entionisiertem Wasser auf insgesamt 100 Gewichtsteile verdünnt sind.

16. Haarkosmetisches Mittel nach Anspruch 15, dadurch gekennzeichnet, dass sie mit einer wässrigen Lösung aus Natriumhydroxyd oder Zitronensäure auf einen pH-Wert von 5 bis 8 eingestellt sind.

17. Haarbehandlungsverfahren, dadurch gekennzeichnet, dass man ein kosmetisches Mittel gemäss einem der Ansprüche 14 bis 16 auf mit Leitungswasser angefeuchtetem Haar bei 20 bis 40°C aufbringt, das Haar schampomiert und konditioniert.

**Claims**

1. A copolymeric, quaternary ammonium salt which is soluble or forms a micro emulsion in an aqueous surfactant system, which salt has a molecular weight distribution of $10^4$ to $10^9$, the molecular weight of at least 5 per cent by weight of the copolymer being $10^7$ to $10^9$, and contains, in any order, on average 5 to 80 mol% of recurring structural elements of the formula

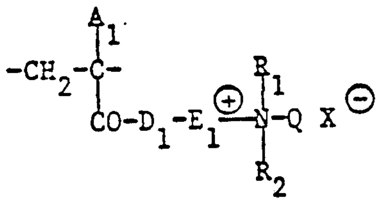 ,

on average 10 to 95 mol% of recurring structural elements of the formula

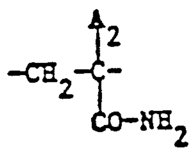

and 0 to on average 10 mol% of structural elements of the formula

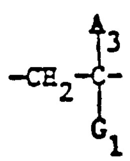 and optionally

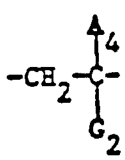

in which formulae $A_1$, $A_2$, $A_3$ and $A_4$ are each hydrogen or methyl, $G_1$ and $G_2$ differ from one another and are each -CN, -COOH or $-CO-D_2-E_2-$

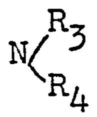 ,

$D_1$ and $D_2$ are each oxygen or -NH-, $E_1$ and $E_2$ are each alkylene having 1 to 4 carbon atoms and which is unsubstituted or substituted by hydroxy, $R_1$, $R_2$, $R_3$ and $R_4$ are each methyl or ethyl, Q is alkyl or hydroxyalkyl having 1 to 4 carbon atoms or benzyl and $X^{\ominus}$ is the radical of at least one anionic surfactant, or at least one anionic surfactant which has zwitterion character.

2. An ammonium salt according to claim 1, which can be obtained by water-in-oil emulsion polymerisation or a solution polymerisation of a quaternary ammonium salt of the acrylic acid series and at least one other acrylic comonomer, isolation of the copolymer and reaction thereof with an anionic surfactant, or an anionic surfactant which has zwitterion character, ion exchange taking place.

3. An ammonium salt according to either of claims 1 or 2, in which the molecular weight of 5 to 60 per cent by weight of the copolymer is $10^7$ to $10^9$.

4. An ammonium salt according to any one of claims 1 to 3, in which $X^{\ominus}$ in the formula given in claim 1 is the radical of a surface-active sarcosinate, sulfate (sulfate-ether), sulfonate or sulfosuccinic acid derivative, fluorinated surfactant or phosphate surfactant.

5. An ammonium salt according to any one of claims I to 3, in which $X^{\ominus}$ in the formula given in claim 1 is the radical of a surface-active N-alkyl-α-iminodipropionate which has zwitterion character, or of an imidazoliniumdicarboxylic acid derivative substituted in the 2-position by alkyl.

6. A process for the preparation of an ammonium salt according to any one of claims 1 to 5, in which the comonomers of the formulae

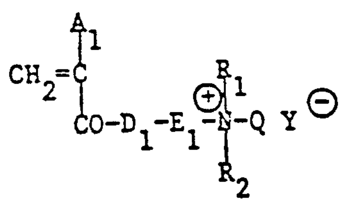 ,

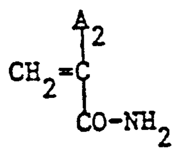 ,

and optionally

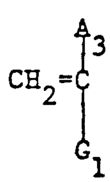

and optionally

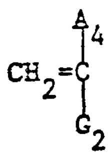

in which formulae $A_1$, $A_2$, $A_3$, $A_4$, $D_1$, $E_1$, $G_1$, $G_2$, $R_1$, $R_2$ and Q are as defined in claim 1 and $Y^{\ominus}$ is a halide anion or an alkyl-sulfate or alkyl-phosphonate anion having 1 to 4 carbon atoms in the alkyl radical, are copolymerised by water-in-oil emulsion polymerisation in the presence of a water-in-oil emulsifier and, optionally, an emulsion stabiliser, or by solution polymerisation, in each case in the presence of a polymerisation initiator, and the copolymer is precipitated with a water-soluble-solvent and is then dried, which process comprises reacting the resultant copolymer with at least one anionic surfactant, or at least one anionic surfactant which has zwitterion character of the formula

$X^{\ominus} Z^{\oplus}$

in which $Z^{\oplus}$ is an alkali metal cation or an ammonium cation which is unsubstituted or substituted by alkyl or alkanol radicals each having 1 to 4 carbon atoms and $X^{\ominus}$ is as defined in claim 1, in an aqueous medium at 10 to 60°C and at a pH value of 5 to 9, the anion $Y^{\ominus}$ being replaced by the anion $X^{\ominus}$.

7. A process according to claim 6, wherein the reaction with ion exchange is carried out in the temperature range from 15 to 40°C for 30 to 100 minutes.

8. A process according to either of claims 6 or 7, which comprises the use of an excess of at least one anionic surfactant,or at least one anionic surfactant which has zwitterion character of the formula according to claim 6, based on the monomeric, quaternary ammonium salt used in the preparation of the copolymer.

9. A process according to claim 8, wherein 4 to 500 mol of surfactant are employed per mol of monomeric ammonium salt.

10. Use of a polymeric ammonium salt according to any one of claims 1 to 5 in a cosmetic.

11. Use according to claim 10, wherein the polymeric ammonium salt is employed in a hair cosmetic.

12. A cosmetic, which contains at least one polymeric ammonium salt according to any one of claims 1 to 5.

13. A cosmetic according to claim 12, which contains an excess of at least one of the anionic surfactants,or at least one of the anionic surfactants which have zwitterion character of the formula according to claim 6, used in the preparation of the ammonium salt.

14. A cosmetic according to claim 13, which is a hair cosmetic.

15. A hair cosmetic according to claim 14, which contains 0.05 to 1.5 parts by weight, calculated as active ingredient, of at least one polymeric ammonium salt according to any one of claims 1 to 5, 5 to 20 parts by weight, calculated as active ingredient, of at least one anionic surfactant, or at least one anionic surfactant which has zwitterion character, and, optionally, a cosmetic assistant and which cosmetic is diluted with demineralised water to a total of 100 parts by weight.

16. A hair cosmetic according to claim 15, which is adjusted to a pH value of 5 to 8 with an aqueous solution of sodium hydroxide or citric acid.

17. Hair treatment method, which comprises applying a cosmetic according to any one of claims 14 to 16 to hair, which has been wetted with tapwater, at 20 to 40°C and shampooing and conditioning the hair.

**Revendications**

1. Sels d'ammonium quaternaire copolymères, solubles ou micro-émulsifiables dans des systèmes tensioactifs aqueux, caractérisés en ce qu'ils présentent une distribution des masses moléculaires allant de $10^4$ à $10^9$, la masse moléculaire d'au moins 5 % en poids du copolymère allant de $10^7$ à $10^9$, et en ce qu'ils contiennent, dans un ordre quelconque, en moyenne 5 à 80 mole % de motifs structuraux répondant à la formule

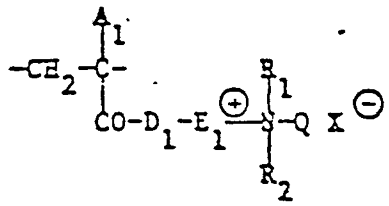
,

en moyenne 10 à 95 mole % de motifs structuraux répondant à la formule

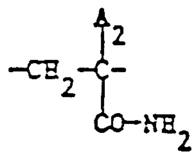

et

0 à en moyenne 10 mole % de motifs structuraux répondant aux formules

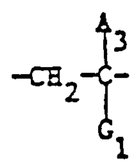

et éventuellement

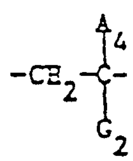
.

dans lesquelles $A_1$, $A_2$, $A_3$ et $A_4$ désignent chacun l'hydrogène ou un groupe méthyle, $G_1$ et $G_2$ sont différents l'un de l'autre et désignent chacun -CN -COOH ou -CO-D -$E_2$

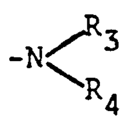
,

$D_1$ et $D_2$ désignent chacun l'oxygéne ou -NH-, $E_1$ et $E_2$ sont chacun un alkylène en $C_1$ à $C_4$, qui est substitué le cas échéant par un hydroxyle, $R_1$, $R_2$, $R_3$ et $R_4$ désignent chacun un groupe méthyle ou éthyle, Q est un groupe alkyle ou hydroxyalkyle en $C_1$ à $C_4$ ou benzyle, et $X^{\ominus}$ est le résidu d'au moins un agent tensioactif ou d'un agent tensioactif anionique amphotère.

2. Sels d'ammonium conformes à la revendication 1, caractérisés en ce qu'on peut les obtenir par polymérisation en émulsion eau-dans-huile ou par polymérisation en solution d'un sel d'ammonium quaternaire de la série de l'acide acrylique et d'au moins un autre comonomère à base acrylique, isolement du copolymère et réaction par échange d'ions avec un agent tensioactif anionique ou un agent tensioactif anionique amphotère.

3. Sels d'ammonium conformes à l'une des revendications 1 ou 2, caractérisés en ce que la masse moléculaire de 5 à 60 % en poids du copolymère s'élève à des valeurs allant de $10^7$ à $10^9$.

4. Sels d'ammonium conformes à l'une des revendications 1 à 3, caractérisés en ce que $X^{\ominus}$, dans la formule indiquée dans la revendication 1, représente le résidu d'un sarcosinate, sulfate (éthersulfate), sulfonate, ou

dérivé sulfosuccinate tensioactif, d'un agent tensioactif fluoré ou d'un agent tensioactif phosphate.

5. Sels d'ammonium conformes à l'une des revendications 1 à 3, caractérisés en ce que $X^{\ominus}$, dans la formule indiquée dans la revendication 1, représente le résidu d'un N-alkyl-α-iminodipropionate tensioactif amphotère, ou d'un dérivé acide imidazoliniumdicarboxylique substitué en position 2 par un groupe alkyle.

6. Procédé de préparation d'un sel d'ammonium conforme à l'une des revendications 1 à 5, dans lequel on effectue la copolymérisation des comonomères de formule

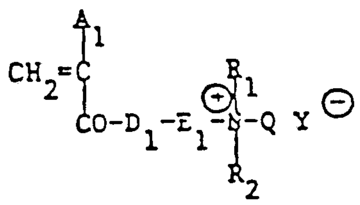
,

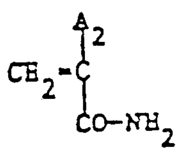
,

éventuellement

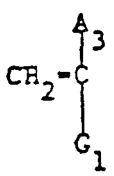

et éventuellement

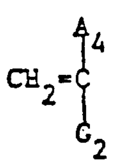
,

formules dans lesquelles $A_1$, $A_2$, $A_3$, $A_4$, $D_1$, $E_1$, $G_1$, $G_2$, $R_1$, $R_2$ et Q ont les significations indiquées dans la revendication 1, et $Y^{\ominus}$ représente un anion halogénure, alkylsulfate ou alkylphosphonate, présentant de 1 à 4 atomes de carbone dans la partie alkyle, par polymérisation en émulsion eau-dans-huile en présence d'un émulsionnant eau-dans-huile et éventuellement d'un stabilisant d'émulsion, ou bien par polymérisation en solution, dans chaque cas en présence d'un agent d'amorçage de la polymérisation, on précipite le copolymère dans un solvant soluble dans l'eau, et on le sèche ensuite, caractérisé en ce que l'on fait réagir le copolymère ainsi obtenu avec au moins un agent tensioactif anionique ou un agent tensioactif anionique amphotère, de formule

$X^{\ominus} Z^{\oplus}$,

dans laquelle $Z^{\oplus}$ est un cation alcalin ou un cation ammonium éventuellement substitué par des radicaux alkyle ou alcanol en $C_{1-4}$ et $X^{\ominus}$ a la signification indiquée dans la revendication 1, en milieu aqueux, à une température de 10 à 60°C, à une valeur de pH de 5 à 9, par échange de l'anion $Y^{\ominus}$ avec l'anion $X^{\ominus}$.

7. Procédé conforme à la revendication 6, caractérisé en ce que l'on effectue la réaction d'échange d'ions à une température allant de 15 à 40°C pendant 30 à 100 minutes.

8. Procédé selon l'une des revendications 6 et 7, caractérisé en ce que l'on utilise un excès d'au moins un agent tensioactif anionique ou un agent tensioactif anionique amphotère de la formule indiquée dans la revendication 6, par rapport aux sels d'ammonium quaternaire monomères utilisés pour la préparation du copolymère.

9. Procédé conforme à la revendication 8, caractérisé en ce que l'on utilise de 4 à 500 moles d'agent

tensioactif par mole de sel d'ammonium monomère.

10. Utilisation des sels d'ammonium polymères conformes à l'une des revendications 1 à 5 dans des cosmétiques.

11. Utilisation conforme à la revendication 10, caractérisée en ce que les sels d'ammonium polymères sont utilisés dans des cosmétiques capillaires.

12. Cosmétiques caractérisés en ce qu'ils contiennent un sel d'ammonium polymère conforme à l'une des revendications 1 à 5.

13. Cosmétiques conformes à la revendication 12, caractérisés en ce qu'ils contiennent un excès d'au moins un des agents tensioactifs anionique ou anionique amphotère de formule indiquée dans la revendication 6, utilisés pour la préparation du sel d'ammonium.

14. Cosmétiques conformes à la revendication 13, caractérisés en ce que ce sont des cosmétiques capillaires.

15. Cosmétiques capillaires conformes à la revendication 14, caractérisés en ce qu'ils contiennent de 0,05 à 1,5 partie en poids, calculée en substances actives, d'au moins un sel d'ammonium polymère conforme à l'une des revendications 1 à 5, de 5 à 20 parties en poids, calculées en substances actives, d'au moins un agent tensioactif anionique ou un agent tensioactif anionique amphotère, et éventuellement des adjuvants cosmétiques, et en ce qu'ils sont dilués à 100 parties en poids au total avec de l'eau désionisée.

16. Cosmétiques capillaires conformes à la revendication 15, caractérisés en ce qu'ils sont ajustés à une valeur de pH allant de 5 à 8 à l'aide d'une solution aqueuse d'hydroxyde de sodium ou d'acide citrique.

17. Procédé de traitement de cheveux, caractérisé en ce que l'on applique un cosmétique conforme à l'une des revendications 14 à 16 sur une chevelure mouillée à l'eau du robinet, on effectue un shampoing de la chevelure et on la conditionne.